# EUROPEAN PATENT SPECIFICATION

(11) **EP 3 009 519 B1**
(45) Date of publication and mention of the grant of the patent: **03.10.2018**
(21) Application number: 15189671.9
(22) Date of filing: 14.10.2015
(51) Int. Cl.: C12Q 1/58, B01L 3/00, G01N 33/487, G01N 33/52, G01N 33/62, G01N 33/84

(54) **TEST DEVICE, REACTIVE ELEMENT AND ARRANGEMENT FOR UREASE ACTIVITY**
TESTVORRICHTUNG, REAKTIVES ELEMENT UND ANORDNUNG FÜR UREASEAKTIVITÄT
DISPOSITIF DE TEST, ÉLÉMENT RÉACTIF ET AGENCEMENT POUR ACTIVITÉ DE L'URÉASE

(30) Priority: 14.10.2014 FI 20145897
(43) Date of publication of application: 20.04.2016
(73) Proprietor: AMA-Med Oy, 50130 Mikkeli (FI)
(72) Inventor: DMITRIENKO, Marina, St. Petersburg 198215 (RU); DMITRIENKO, Vadim, St. Petersburg 198215 (RU); KORNIENKO, Elena, St. Petersburg 192029 (RU); KOLOMINA, Elena, St. Petersburg 195297 (RU)
(74) Representative: Berggren Oy, Helsinki & Oulu

(56) References cited:
- EP-A2- 0 454 046
- WO-A1-93/19200
- WO-A1-2012/054545
- WO-A2-2011/112106
- JP-A- 2006 193 477
- SU-A1- 1 507 797
- US-A- 5 198 335
- US-A1- 2010 028 937

## Description

This invention relates to a test device usable in analysis of urease activity in a sample, a reactive element usable in said device, a method for preparing said element and an arrangement for an automated analysis of urease activity.

### Background

More than 50% of the world's population harbor *Helicobacter pylori* (previously named *Campylobacter pylori*) in their upper gastrointestinal tract. Colonization with *H. pylori* is not a disease itself but a condition associated with a number of disorders of the upper gastrointestinal tract. Individuals infected with *H. pylori* have a lifetime risk of developing peptic ulcers and a risk of acquiring stomach cancer. *Helicobacter pylori* is a bacteria commonly associated with duodenal ulcers, stomach cancer and inflammations inside stomach (gastritis).

There are several methods to test *H. pylori* including noninvasively for *H. pylori* infection with a blood antibody test, stool antigen test, or with the carbon urea breath test. However, the most reliable method for detecting *H. pylori* infection is a biopsy check during endoscopy with a rapid urease test, histological examination, and microbial culture. In stomach *H. pylori* neutralizes the acid by producing large amounts of urease, which breaks down the urea present in the stomach to carbon dioxide and ammonia. Urease tests benefit this phenomenon. Determination of urease activity in biopsy is usually based on the color change of the indicator in the medium alkalization with ammonia, formed by hydrolysis of urea.

WO1993019200 discloses a multilayer test device for detection urease in biological tissue specimens comprising matrix containing substrate for urease, a membrane permeable to ammonia and impermeable to water, matrix containing means for detecting ammonia and means for placing the tissue specimen between the substrate element and the diffusion element. Also a kit useful in urease detection containing the multilayer test device and rehydrating solution is proposed.

Commercially available PyloriTek® Reagent Strips (Serim) contain, in separate dry reagent matrices, the urea substrate and a pH indicator covered by a semipermeable membrane. Substrate must be hydrated just prior to performing the test. The result is visually evaluated as a color change of the indicator.

AMA RUT urease test kit (Association of Medicine and Analytics) is based on the color change of the indicator after the biopsy specimen has been placed on the reactive pad. The result is readable within 3 minutes and no separate hydration is needed. Result is visually evaluated.

There is still a need for improved device for analyzing the biopsy samples; especially there is a need for test devices that would allow optical reading and still be easy to use.

### Short description of the invention

Unlike most of the rapid urease tests, this invention detects the urease that is present in the biopsy specimen at the time it is taken, not the urease that can be produced hours later. In addition the test allows automated/digital reading. The first aspect of the invention is a test device. Characteristic features of the device are set forth in claim 1.

The second aspect of the invention is a reactive element. Characteristic features of the reactive element are set forth in claim 9.

The third aspect of the invention is a method of preparing a reactive element. Characteristic features of the method are set forth in claim 11.

The fourth aspect of the invention is an arrangement for an automated analysis of urease activity in a sample, preferably a biopsy sample. Characteristic features of the arrangement are set forth in claim 12.

The invention provides quick and accurate test device for analyzing Helicobacter infection. It also minimizes the amounts of the reagents and factory-made materials, which could introduce an extra error.

### Short description of the drawings

**Figure 1** is a schematic view of a frame for test device.
**Figure 2** **A, B, C** show detail drawings of a reactive element placed in a hollow of the cassette.
**Figure 3** is a top view of a cassette of the device of Fig. 1 without a reactive element and cover film. Values 76.6 and 2.85 (millimeters) are non-restrictive dimensions of one embodiment of the invention.
**Figure 4** shows a longitudinal view of the frame cassette of the device of Fig. 1 without a reactive element and cover film.
**Figure 5** shows a sectional view of a test device with a reactive element, cover film and added biopsy specimen.
**Figure 6** is an example of the result of reading by spectrometer OceanOpctic USB4H00215.
**Figure 7** is a black-and-white photograph the result of the test to be visually evaluated or read by an optical reader.

### Detailed description

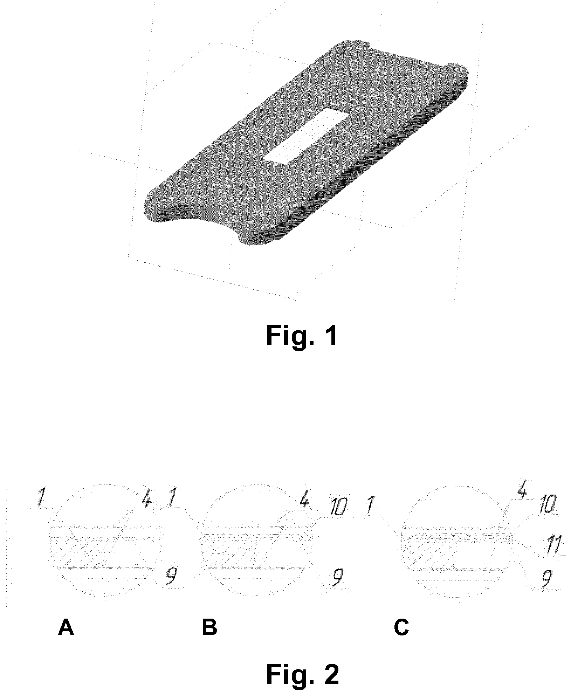

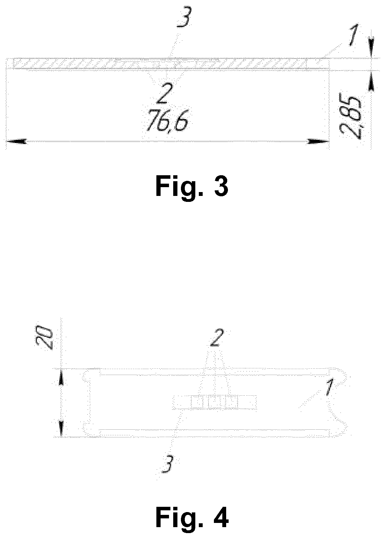

This invention relates to test device suitable for analyzing biopsy samples. The device comprises
(a) a reactive element 5 comprising urea and indicator (Fig. 2); and
(b) a frame cassette 1 (shown in Fig. 3 and Fig. 4 with two hollows 2 and 3).

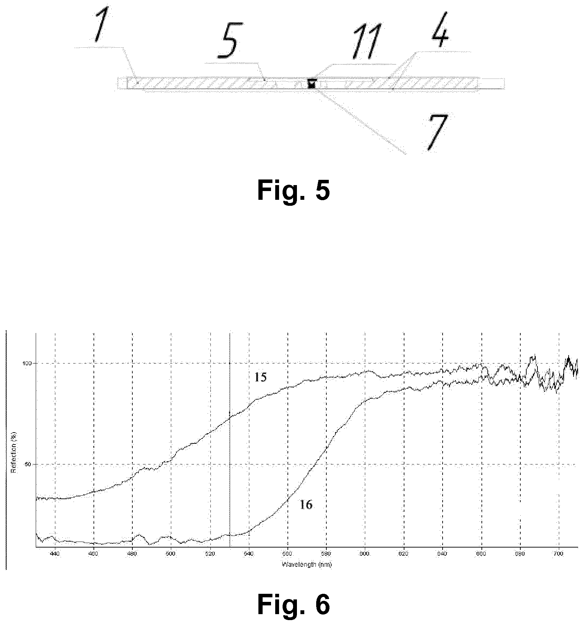

The biopsy specimen should be placed in hollow(s) 2. Hollow 3 is a window for positioning of the reactive element and reading of the result of the test. Hollow for a sample is also called a "well" and can be divided to form two or more wells for biopsy samples. The second side (opposite to the sample) of said reactive element is at least partially open in order to allow evaluation without removal the sample as shown in Fig. 5.

The construction of the device Fig.1 allows interpretation by colorimetric, spectroscopic, photo recorder or other devices without need of removal the sample. Preferably the device (especially the frame) is adapted to fit the desired reading equipment and color (light or dark) of the frame is used to calibrate the optic system). Also white surface of frame can be used by specialist for letter any information about patient. Optical reading will also be enhanced by the reactive element suitable for the device. The reactive element will be discussed later.

For the protection of reactive element from unfavorable factors as humidity, dirt, airing of NH₃ a (preferably transparent) polymeric cover film 4 in Fig. 1, 5 was utilized. Transparent cover film allows reading the result without removal the film. It is also advantageous to cover the wells for the sample using a removable and optionally resealable film. Cover films will also protect the user and reading devices from contamination by the sample. Alternatively the whole device can be stored in a closed container, e.g. a plastic bag or metal film.

The wells 2 in Fig. 3 and 4 for sample enhance the positioning the biopsies. Reasonable small size of the wells allows a reaction without need of hydration of the reactive element and reduces dilution of the urease from the sample. This results in brighter color of indicator and sharp contours thereby improving readability of the test. High speed of reaction is explained by absence of an extra hydration. There is no dilution of urease as the reaction goes in a small volume of biopsy juice.

The wells should have diameter of less than 8 mm, preferably less than 6 mm, most preferably about 4 mm. Preferably each test device has two or three wells to allow parallel analysis of several biopsy assays of each patient.

A reactive element can be single layered Fig. 2A, two layered Fig. 2B or multi layered Fig. 2C

Fig. 2A shows one simple embodiment of the reactive element comprising a hydrophilic material 7 with urea (substrate) and an indicator for producing a color change as a response to changing pH. All the components are included in one layer. There is no need for NH₃ to overcome obstacles during the transition from substrate pad to indicator pad.

Hydrophilic material in a reactive element introduces volume for the reaction medium, matrix for reagents; in case of multilayer system it absorbs the biopsy juice. In one embodiment hydrophilic material is replaced by hydrophobic material impregnated by urea. That material does not absorb the biopsy juice thereby urease from samples is not spreading over surface and it is concentrated on one spot. In that case concentration of NH₃ is higher than in the case of using the hydrophilic membrane.

When urease (of the sample) reacts with urea (substrate) and generates ammonia it is detected by color change of the indicator. The element can be prepared by impregnating a hydrophilic support material, e.g. porous paper or membrane in a solution comprising a substrate and the pH indicating agent (indicator). Impregnation solution may contain also buffer and/or other components such as preservative compounds, antioxidants and etc.

The concentration of urea substrate in a reactive element is preferably adjusted to pH suitable for urease activity. The pH optimum for *H. pylori* surface urease is 7.4 and for intra-bacterial is 5.5 [Cesareo, S. et al. Properties of H. pylori urease compared with Jack Bean Urease 99-15 (1992)]. Suitable pH is thus between 5.5 and 7.5.

The pH indicator should preferably produce a bright color, preferably blue, purple or red (instead of yellowish or greenish) as a response to changing pH. Indicator can be selected from indicators Bromothymol blue (BTB), Phenol red (PR), mixed indicators such as BTB and PR, PR and Bromocresol Purple (BCP) or BTB and PR and BCP, without limiting to those.

One preferred embodiment is shown in Fig. 2B. Preferably the reactive element comprises a (hydrophilic) substrate layer comprising urea on the sample side of the element and an indicator layer 9 capable of producing a color change as a response to changing pH. The first layer (substrate layer) is composed of hydrophilic material which incorporates substrate for reaction (urea). The urease of the specimen places on said layer hydrolyzes urea to ammonia. The generated ammonia penetrates to the second layer (indicator layer) placed on the opposite side of the sample and reacts with the second part of the system.

Advantages of two-layered or multi-layered systems: The combination of hydrophilic layer (as substrate part in multi-layer system) with an indicator layer provides the results without an extra hydration and/or waiting. Using hydrophilic layer with high quantity of urea helps generate NH₃ in necessary amounts. Thickness of indicator layer is typically between 40 and 80 µm, preferably 45 to 60 µm, most preferably about 50 µm. A thin indicator layer avoids spreading the indicator color thereby enhancing the readability. In addition thin layer reacts fast and is sensitive to pH changes.

Preferably the indicator layer is PVA or other film forming polymer layer comprising the pH indicator capable of changing color. PVA film acts as a matrix for pH-indicator and traces of water and provides necessary conditions for solution of indicators and color changing. PVA film is a transparent and thin material. There is no need of waiting when NH₃ passes through all indicator layers as we can see what happens on the border between the layers.

The reactive element further comprises a semipermeable membrane between the substrate layer and the indicator layer Fig. 2C. The membrane should be permeable for ammonia and impermeable for biological liquids and water. Semipermeable material (variable) 10 helps positioning the layers and increases the specificity by the dividing two reactions. Thus it raises readability of the test. Suitable membranes are hydrophobic polymeric materials such as PTFE, PE and others.

Said reactive layer may also be separated from said indicator layer by space. The reactive element can have layers fixed to each other by the frame, using adhesive, by laminating, by plotting or painting. Optionally the indicator layer is painted on the back side (side opposite to the sample) of the substrate layer or the semipermeable layer. Preferably a liquid PVA containing the indicator is used. Plotting or painting allows a continuous contact with the substrate layer thereby increasing the sensitivity of the test. In addition painted surface is smooth which also enhances detection both visually and digitally as the contact is continuous without e.g. air bubbles and the structure is thin.

Optionally a dried PVA film is attached to cover by the adhesive or using the frame cassette. That is a method of fixing of the colored indicator film.

According to an optional embodiment the semipermeable membrane has two sides: hydrophobic and hydrophilic. The hydrophobic side is impregnated with a solution of urea. Then the indicator layer can be spread over the hydrophobic side, e.g. in form of liquid PVA containing the indicator.

Also a "protection film" or "cover film" can be utilized as protection of the device during the storage and the test procedure.

Also a method for preparing a reactive element is within the scope of this invention. The method comprises the steps of
(a) impregnating a hydrophilic support material in solution of urea to provide a substrate layer; and
(b) providing an indicator
   a. by impregnating said support in solution of indicator; or
   b. providing the indicator in form of a separate layer; or
   c. by painting the indicator as a solution of PVA on one side (opposite to sample side) of the substrate layer or the semipermeable membrane.

The methods of the preparation are further explained in the experimental part of the present application.

The result of the test using the test device of the invention is preferably read from the back side of the indicator layer providing the best contrast of color. It has been surprisingly found that smooth back surface and bright and sharp indicator color of the test according to the invention allow digital/automated detection.

This invention is also related to an arrangement for an automated analysis of urease activity in a sample. The arrangement comprises
(a) a test device as described here; and
(b) an automatic detecting device located on the second side of the reactive element.

The reading device can be a colorimetric reader, a spectroscopic reader or a photo recorder, without limiting to those.

The combination the test device of the invention with digital reading assists with interpretation of the test in the event that the result was ambiguous based on visual evaluation. Using automatic reading helps avoiding the subjectivity of the human perception of color and even allows a follow-up of a treatment. Preferably the reactive element in the test device is composed of at least two layers. Layered structure of the reactive element provides an optimal color change and resolution.

A non-limiting example of using the test device according to the present invention is given below with references to Figure 1.
- Remove the cover film 4 from the test-cassette 1. During endoscopy take biopsies specimen from the stomach lining. Put a biopsy 7 specimen on a biopsy well 2. Overlay by the cover film the test cassette 1 with placed biopsy 7.
- Wait for 3 minutes. Turn over a cassette 1.
- Determine the color change on the indicator film 9.
- To improve the accuracy of the result can be used a reading device.

Embodiments of the present disclosure may be illustrated by the following non-limiting examples. It should be understood, however, that the embodiments given in the description above and in the examples are for illustrative purposes only, and that various changes and modifications are possible within the scope of the present disclosure.

### Examples

### Example 1. Single-layer system

Material & reagents: hydrophilic material (chromatographic paper F1 by Munktell & filtrak GmbH or hydrophilic membranes PESHAUEA1 with pore size 1.2 by Cobetter® Filtration), Indicator (Bromothymol blue(BTB), Bromocresol Purple (BCP), Phenol red (PSP), urea (USP urea, Merck), distilled water (pH 5.8-6.4).

Preparation: Indicator and urea were dissolved in water. Sheets of hydrophilic material were dipped into impregnation solution (urea+water+indicator). Impregnation time for paper was 5 to 10 seconds and for PES 10 to 15 seconds.

### Example 2. Two-layer system.

There are two layers:
(1) A substrate part (layer) and
(2) An indicator part (layer)

### Substrate layer

Material & reagents: hydrophilic material (factory-made chromatographic paper or hydrophilic membranes PESHAUEA1 with pore size 1.2 by Cobetter® Filtration, urea (USP urea by Merck), distilled water (pH 5.8-6.4).
Preparation: 18-36 g of Urea and 0.5-1 g of pH indicator were dissolved in 100 ml water. Sheets of hydrophilic material (14x14 mm) were dipped into impregnation solution (urea+water).

### Indicator layer

Material & reagents: PVA (powder), Indicator: (Bromothymol blue(BTB), Bromocresol Purple (BCP), Phenol red (PSP),
Preparation: PVA powder was dissolved in water heated to 100 °C than PVA solution (10% PVA and 90% water) was mixed with water solution of pH indicator (0.2% indicator and 99.8% water). Mixture was spread over smooth surface and was dried to thin film.

### Example 3.

There are two layers:
(1) A substrate part (layer)
(2) An indicator part (layer)

### Substrate layer

Material & reagents: like Example in 2
Preparation: Like in Example 2.

### Indicator layer

Material & reagents: PVA (powder), Indicator: (Bromothymol blue (BTB), Bromocresol Purple (BCP), Phenol red (PSP)., ethyl alcohol, isopropyl alcohol and glycerol.
Preparation: PVA powder was dissolved in water heated to 100 °C than PVA solution (10% PVA and 90% water) was mixed with ethyl alcohol, isopropyl alcohol and glycerol and powder pH indicator. (Mixture of alcohols promotes high accelerating of drying). Mixture was spread over smooth surface and was dried to thin film.

### Example 4.

There are two layers:
(1) A substrate part (layer)
(2) An indicator part (layer)

### Substrate layer

Material & reagents: like in Example 2
Preparation: Like in Example 2.

### Indicator layer

Material & reagents: adhesive (UV adhesive UCA by N.V. UNICO or heat activated adhesive KIWOTERM L 110 by KIWO®), Indicator: (Bromothymol blue(BTB), Bromocresol Purple (BCP), Phenol red (PSP)., ethyl alcohol, acetone.
Preparation: adhesive (UV adhesive UCA by N.V. UNICO or heat activated adhesive KIWOTERM L 110 by KIWOO) was mixed with ethyl-acetone solution of the indicator. Mixture was spread over surface of the cover film of the test kit and was died according to adhesive specification enclosed.

### Example 5.

There are two layers:
(1) A substrate part (layer)
(2) An indicator part (layer)

### Substrate layer

Material & reagents: Semi permeable membrane (factory-made membrane PTFE by Cobetter® Filtration), urea, ethyl alcohol, distilled water (pH 5.8-6.4).
Preparation: 18-36 g of Urea and 0.5-1 g of pH indicator were dissolved in 50 ml water. Solution was mixed with 50 ml ethyl alcohol. Sheets of material (14x14 mm) were dipped into impregnation solution (urea+water+ethyl alcohol).

### Indicator layer

Material & reagents: Like in Example 3.
Preparation: Like in Example 3

### Example 6.

Example 6. has similar composition as Example 2. Extra component is hydrophobic film.

Difference: Mixture of PVA, water, indicator was applied to a hydrophobic film than was dried and PVA film was firmly fixed on a hydrophobic film.

### Example 7.

This system consists of three layers:
(1) A sensitive part (layer)
(2) A semipermeable membrane
(3) An indicator part (layer)

Parts (1) and (3) were like parts (1) and (2) in Example 2. Semi permeable membrane was factory-made membrane PTFE by Cobetter® Filtration). All parts of were separately placed into the cassette.

### Example 8.

This system consists of three layers:
(1) A sensitive part (layer)
(2) A semipermeable membrane
(3) An indicator part (layer)

Parts (1) and (3) were like parts (1) and (2) in Example 2. Semi permeable membrane was factory-made membrane PTFE by Cobetter® Filtration). Difference to Example 6: PVA solution (with pH indicator) spread over hydrophobic membranes and was dried.

### Example 9. Fixed two sided system

Material & reagents: two-sides (one side is hydrophilic and another side is hydrophobic) material (factory-made material GOTF-3 and GOTF-5 by Cobetter® Filtration), Indicator: (Bromothymol blue(BTB), Bromocresol Purple (BCP), Phenol red (PSP), urea (USP urea by Merck), distilled water (with pH 5.8-6.4), ethyl alcohol.
Preparation: Urea was dissolved in water. Membrane was dipped into solution and dried. Over dried membrane was spread PVA solution (PVA, indicator, water).

### Example 10. Assembling the device

All types of reactive element were cut into rectangle 24*4 mm and placed into the cavity in the frame cassette as shown in Figure 1 (cassette) on the order of description in part Ex.

Then the cassette with the sensitive element was covered (laminated) by a PVC cover film.

### Example 11. Testing the devices

The test devices as described in Examples 1 to 9 were used in testing biopsy samples known to be positive and samples known to be negative. All the reactive elements produced result that was clearly visible from side opposite to the sample.

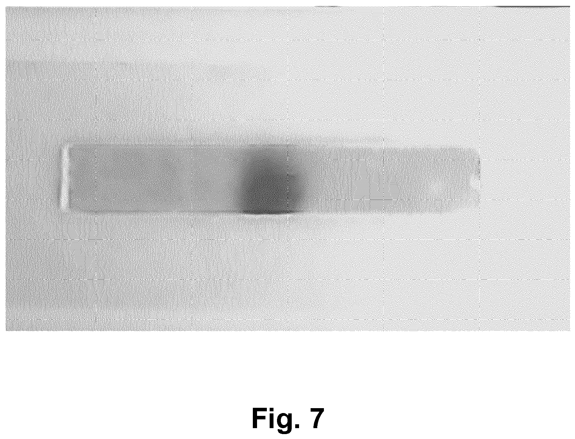

The samples with various reactive elements were read using:
1. Spectrometer OceanOpctic USB4H00215. Reactive elements according to Examples 2 to 9 provided excellent reading results. Data of reading spectrometer OceanOpctic USB4H00215 is shown as Figure 6. The line 15 in Fig.6 shows a yellow color (not visible in black-and-white photograph shown as Fig. 7) which accords to color of a sensitive element before analysis or a result was negative. The line 16 in Fig.6. shows a red color (originally red color can be seen as a darker area in black-and-white photograph shown as Fig. 7) according a positive reaction. The result is difference between yellow and red lines.
2. Photorecorder CHR-310, Kaiwood Technology Co., Ltd.

Figure 7 shows a black-and-white photograph of the indicator layer of the test device. Reading result of this test is shown as Figure 6. The software of the device determines the areas of color changing and gives a result.

## Claims

1. A test device comprising
(a) a reactive element comprising a substrate layer comprising urea on the sample side of the element and an indicator layer; and
(b) a frame with at least one well for sample to be placed on the first side of said reactive element,
wherein the second side (opposite to the sample) of said reactive element is at least partially open in order to allow evaluation without removal the sample and that the indicator layer is a PVA layer comprising the pH indicator and wherein the substrate layer is a semipermeable membrane

2. The device of claim 1, wherein said semipermeable membrane has hydrophobic and hydrophilic sides.

3. The device of claim 1 or 2, wherein said PVA film comprising the indicator is painted on said semipermeable film or hydrophobic material.

4. The device of any of the preceding claims, wherein the indicator layer is an adhesive comprising the pH indicator.

5. The device of any of the preceding claims, wherein the layers are attached to each other by an adhesive.

6. The device of any of the preceding claims, wherein said reactive element is covered with a removable or transparent layer.

7. The device of any of the preceding claims, **characterized in that** said hydrophobic semipermeable membrane is PTFE or PE, preferably PTFE.

8. The device of any of claims 1 to 7, **characterized in that** said well(s) for sample has (have) a diameter of less than 8 mm.

9. A reactive element comprising a hydrophilic substrate layer comprising urea on the sample side of the element and an indicator layer, wherein said indicator layer comprises PVA and a pH indicator and wherein said reactive element further comprises a semipermeable membrane between the substrate layer and the indicator layer.

10. The reactive element of claim 9, **characterized in that** said substrate layer is a semipermeable membrane.

11. A method for preparing a reactive element according to claim 9 or 10 comprising the steps of
(a) impregnating a support material in solution of urea to provide a substrate layer; and
(b) providing an indicator layer; and
(c) providing a semipermeable membrane between the substrate layer and the indicator layer.

12. An arrangement for an automated analysis of urease activity in a sample comprising
(a) a test device of any of claims 1 to 8; and
(b) an automatic detecting device located on the second side of the reactive element.

13. The arrangement of claim 12, wherein the detecting system is a colorimetric reader, a spectroscopic reader or a photo recorder.

## Patentansprüche

1. Testvorrichtung, umfassend
(a) ein reaktives Element, das eine Substratschicht umfasst, die Harnstoff auf der Probenseite des Elementes und eine Anzeigeschicht umfasst; und
(b) einen Rahmen mit mindestens einer Wanne (Well) für die Probe, die auf der ersten Seite des reaktiven Elementes platziert werden soll,
wobei die zweite Seite (gegenüber der Probe) des reaktiven Elementes zumindest teilweise offen ist, um eine Bewertung ohne Entfernung der Probe zu ermöglichen, und dass die Anzeigeschicht eine PVA-Schicht ist, die den pH-Indikator enthält, und wobei die Substratschicht eine semipermeable (halbdurchlässige) Membran ist.

2. Vorrichtung nach Anspruch 1, wobei die semipermeable Membran hydrophobe und hydrophile Seiten hat.

3. Vorrichtung nach Anspruch 1 oder 2, wobei der PVA-Film, der den Indikator enthält, auf den semipermeablen Film oder das hydrophobe Material aufgestrichen ist.

4. Vorrichtung nach einem der vorherigen Ansprüche, wobei die Anzeigeschicht ein Klebstoff ist, der den pH-Indikator enthält.

5. Vorrichtung nach einem der vorherigen Ansprüche, wobei die Schichten aneinander durch einen Klebstoff befestigt sind.

6. Vorrichtung nach einem der vorherigen Ansprüche, wobei das reaktive Element mit einer entfernbaren oder transparenten Schicht bedeckt ist.

7. Vorrichtung nach einem der vorherigen Ansprüche, **dadurch gekennzeichnet, dass** die hydrophobe semipermeable Membran PTFE oder PE ist, vorzugsweise PTFE.

8. Vorrichtung nach einem der Ansprüche 1-7, **dadurch gekennzeichnet, dass** die Wanne(n) für die Probe einen Durchmesser von weniger als 8 mm hat (haben).

9. Reaktives Element, das eine hydrophile Substratschicht umfasst, die Harnstoff auf der Probenseite des Elementes enthält und eine Indikatorschicht, wobei die Indikatorschicht PVA und einen pH-Indikator enthält und wobei das reaktive Element ferner eine semipermeable Membran zwischen der Substratschicht und der Indikatorschicht umfasst.

10. Reaktives Element nach Anspruch 9, **dadurch gekennzeichnet, dass** die Substratschicht eine semipermeable Membran ist.

11. Verfahren zum Herstellen eines reaktiven Elementes nach Anspruch 9 oder 10, das die folgenden Schritte umfasst
(a) Imprägnieren eines Stützmaterials in Lösung von Harnstoff, um eine Substratschicht bereitzustellen; und
(b) Bereitstellen einer Indikatorschicht; und
(c) Bereitstellen einer semipermeablen Membran zwischen der Substratschicht und der Indikatorschicht.

12. Anordnung für eine automatische Analyse der Harnstoffaktivität in einer Probe, umfassend
(a) eine Testvorrichtung nach einem der Ansprüche 1 bis 8; und
(b) eine automatische Nachweisvorrichtung, die sich auf der zweiten Seite des reaktiven Elementes befindet.

13. Anordnung nach Anspruch 12, wobei das Nachweissystem ein kolorimetrisches Lesegerät, ein spektroskopisches Lesegerät oder ein Fotorekorder ist.

## Revendications

1. Dispositif de test comprenant
(a) un élément réactif comprenant une couche de substrat comprenant de l'urée sur le côté d'échantillon de l'élément et une couche d'indicateur ; et
(b) un cadre avec au moins un puits a échantillon devant être placé sur le premier côté dudit élément réactif,
dans lequel le second côté (situé à l'opposé de l'échantillon), dudit élément réactif est au moins partiellement ouvert de façon à permettre une évaluation sans l'élimination de l'échantillon et où la couche d'indicateur est une couche de PVA comprenant l'indicateur de pH et dans lequel la couche de substrat est une membrane semiperméable.

2. Dispositif selon la revendication 1, dans lequel ladite membrane semiperméable présente des côtés hydrophobe et hydrophile.

3. Dispositif selon la revendication 1 ou la revendication 2, dans lequel ledit film de PVA comprenant l'indicateur est peint sur ledit film semiperméable ou sur ledit matériau hydrophobe.

4. Dispositif selon l'une quelconque des revendications précédentes, dans lequel la couche d'indicateur est un adhésif comprenant l'indicateur de pH.

5. Dispositif selon l'une quelconque des revendications précédentes, dans lequel les couches sont attachées l'une à l'autre au moyen d'un adhésif.

6. Dispositif selon l'une quelconque des revendications précédentes, dans lequel ledit élément réactif est recouvert d'une couche qui peut être enlevée ou transparente.

7. Dispositif selon l'une quelconque des revendications précédentes, **caractérisé en ce que** ladite membrane semiperméable hydrophobe est du PTFE ou du PE, de préférence du PTFE.

8. Dispositif selon l'une quelconque des revendications 1 à 7, **caractérisé en ce que** ledit puits / lesdits puits pour échantillon possède / possèdent un diamètre inférieur à 8 mm.

9. Élément réactif comprenant une couche de substrat hydrophile comprenant de l'urée sur le côté d'échantillon de l'élément et une couche d'indicateur, dans lequel ladite couche d'indicateur comprend du PVA et un indicateur de pH, et dans lequel ledit élément réactif comprend en outre une membrane semiperméable située entre la couche de substrat et la couche d'indicateur.

10. Élément réactif selon la revendication 9, **caractérisé en ce que** ladite couche de substrat est une membrane semiperméable.

11. Procédé de préparation d'un élément réactif selon la revendication 9 ou la revendication 10, comprenant les étapes de
(a) imprégnation d'un matériau de support dans une solution d'urée pour fournir une couche de substrat ; et
(b) fourniture d'une couche d'indicateur ; et
(c) fourniture d'une membrane semiperméable située entre la couche de substrat et la couche d'indicateur.

12. Agencement destiné à une analyse automatisée d'activité d'uréase dans un échantillon comprenant
(a) un dispositif de test selon l'une quelconque des revendications 1 à 8 ; et
(b) un dispositif de détection automatique situé sur le second côté de l'élément réactif.

13. Agencement selon la revendication 12, dans lequel le système de détection est un lecteur colorimétrique, un lecteur spectroscopique ou un enregistreur photographique.
